# EUROPEAN PATENT SPECIFICATION

(11) **EP 3 857 554 B1**
(45) Date of publication and mention of the grant of the patent: **11.09.2024**
(21) Application number: 19780321.6
(22) Date of filing: 27.09.2019
(51) Int. Cl.: G16B 10/00

(54) **METHOD AND APPARATUS FOR ANALYSING A SAMPLE**
VERFAHREN UND VORRICHTUNG ZUR ANALYSE EINER PROBE
PROCÉDÉ ET APPAREIL POUR L'ANALYSE D'UN ÉCHANTILLON

(30) Priority: 28.09.2018 GB 201815920
(43) Date of publication of application: 04.08.2021
(73) Proprietor: Genome Research Limited, Hinxton, Saffron Walden CB10 1SA (GB)
(72) Inventor: FORSTER, Samuel, Clayton, Victoria VIC 3168 (AU); LAWLEY, Trevor, Cambridge Cambridgeshire CB10 1SA (GB); NEVILLE, Anne, Cambridge Cambridgeshire CB10 1DR (GB)
(74) Representative: Appleyard Lees IP LLP
(86) International application number: PCT/GB2019/052744
(87) International publication number: WO 2020/065347

(56) References cited:
- US-A1- 2015 032 711
- WIEHLMANN LUTZ ET AL: "Impact of sample processing on human airways microbial metagenomes", JOURNAL OF BIOTECHNOLOGY, ELSEVIER, AMSTERDAM, NL, vol. 250, 23 January 2017 (2017-01-23), pages 51 - 55, XP085018857, ISSN: 0168-1656, DOI: 10.1016/J.JBIOTEC.2017.01.001
- PATRICIA MORAN LOSADA ET AL: "The cystic fibrosis lower airways microbial metagenome", ERJ OPEN RESEARCH, vol. 2, no. 2, 13 April 2016 (2016-04-13), pages 00096 - 2015, XP055645126, DOI: 10.1183/23120541.00096-2015
- CHI-CHING LEE ET AL: "Metabolic classification of microbial genomes using functional probes", BMC GENOMICS, BIOMED CENTRAL, vol. 13, no. 1, 27 April 2012 (2012-04-27), pages 157, XP021124636, ISSN: 1471-2164, DOI: 10.1186/1471-2164-13-157

## Description

### Field

The invention relates to a method and apparatus for analysing a sample, e.g. from a patient, for example analysing a faecal sample using metagenomic techniques or analysing a sample for epidemiological purposes.

### Background

A typical human intestinal microbiota contains 100-1000 bacterial species. The majority of the bacterial species within the adult human microbiota are derived from four high level taxonomic classifications or phyla, the Firmicutes, Bacteroidetes, Actinobacteria and Proteobacteria. These groups change in abundance from birth to adulthood to old age, reflecting changing environmental influences such as initial mode of delivery, diet, insults such as pathogen infection and in many cases antibiotic usage (Dominguez-Bello, M. G., E. K. Costello, M. Contreras, M. Magris, G. Hidalgo, N. Fierer and R. Knight (2010) "Delivery mode shapes the acquisition and structure of the initial microbiota across multiple body habitats in newborns." Proc Natl Acad Sci USA 107(26): 11971-11975). In adulthood, the intestinal microbiota is dominated by the Firmicutes and the Bacteroidetes, both of which are strict anaerobes.

The intestinal microbiota plays a key role in digesting food inaccessible to the human gastrointestinal tract, such as metabolizing carbohydrates into short chain fatty acids, interacting with the immune system to maintain homeostasis, promoting maturation of the gut and development of the immune system (Hooper et al (2012) "Interactions between the microbiota and the immune system." Science 336(6086): 1268-1273). The intestinal microbiota also plays an important role in resisting pathogen invasion, termed 'colonisation resistance'. This functions through the diversity and abundance of commensal species present and through the occupation of key niches and utilization of nutrients (Lawley, T. D. and A. W. Walker (2013). "Intestinal colonization resistance." Immunology 138(1): 1-11). If microbial homeostasis is disturbed, for example through use of antibiotics, a shift towards dysbiosis can occur. In dysbiosis or disrupted symbiosis, microbiota functions can be lost or deranged, resulting in increased susceptibility to pathogens, altered metabolic profiles, or induction of proinflammatory signals that can result in local or systemic inflammation or autoimmunity. Dysbiosis occurs when the microbiota contributes to a manifestation or continuation of a given disease that cannot be attributed to a single species (Sommer et al: The resilience of the intestinal microbiota influences health and disease, Nature, Vol 15, pages 630-638, 2017).

Given the evidence that many people harbour multiple strains of the same bacterial species within their gastrointestinal microbiota, there is now a need to improve the precision and accuracy of analysis methods to enable functional validation and the development of microbiome-based therapeutics. As described in "Human Microbiome Project, C. Structure, function and diversity of the healthy human microbiome" published in Nature 486, 207-214, doi:10.1038/nature11234 (2012), the Human Microbiome Project (HMP), has made substantial progress in genome sequencing bacteria isolates derived from across 18 sites in the human body.

With recent advances in bacterial culturing methods in the human gastrointestinal microbiota, it is now possible to efficiently grow and purify the vast majority of these bacteria in the laboratory, for example as described in "Culturing of 'unculturable' human microbiota reveals novel taxa and extensive sporulation" by Browne et al published in Nature 533, 26 May 2016. These comprehensive collections of bacterial genomes provide the ability to perform precise, reference-based metagenomic assembly and analysis (RBMA) and achieve species and strain level taxonomic classification of the bacterial composition. In addition, access to archived bacterial isolates for further phenotypic validation and functional investigation facilitates the advance from sequence-based, correlative studies to causative phenotypic validation of predicted bacterial function as described in "Commensal Koch's postulates: establishing causation in human microbiota research" by Neville et al published in Curr Opin Microbiol 42, 47-52, doi:10.1016/j.mib.2017.10.001 (2017).

Metagenomics sequencing is a central tool to study the microbial compositions and functions from both natural and built environments and is described for example in "Recovery of nearly 8,000 metagenome-assembled genomes substantially expands the tree of life" by Parks et al published in Nat Microbiol 2, 1533-1542, doi:10.1038/s41564-017-0012-7 (2017) or "A human gut microbial gene catalogue established by metagenomic sequencing" by Qin et al published in Nature 464, 59-65, doi:10.1038/nature08821 (2010). The human gastrointestinal microbiota is dominated by bacteria, but may also include viruses, archaea, fungi and other eukaryotes. Amplicon sequencing, targeting the 16S rRNA gene, provides a capacity to determine high level bacterial and archaeal compositions and detect structural changes in microbial communities. However, as illustrated for example in "Control of pathogens and pathobionts by the gut microbiota by Kamada et al published in Nat Immunol 14, 685-690, doi:10.1038/ni.2608 (2013), significant and biologically relevant phenotypic differences exist even between closely phylogenetically related bacterial strains of the same species. Amplicon sequencing may not be useful for differentiating in these cases.

An alternative technique is shotgun metagenomic sequencing which provides an assessment of the entire genomic content of the microbial community (bacteria, viruses, fungi, archaea) and achieves the precise taxonomic classification required for functional understanding. One example is described in "Strains, functions and dynamics in the expanded Human Microbiome Project" by Lloyd-Price et al published in Nature 550, 61-66, doi:10.1038/nature23889 (2017). Unfortunately, these approaches can be expensive, laborious and fundamentally constrained by their requirement for deep sequence coverage and inability to differentiate between closely related bacterial taxa as highlighted in "Shotgun metagenomics, from sampling to analysis" by Quince et al published in Nat Biotechnol 35, 833-844, doi:10.1038/nbt.3935 (2017).

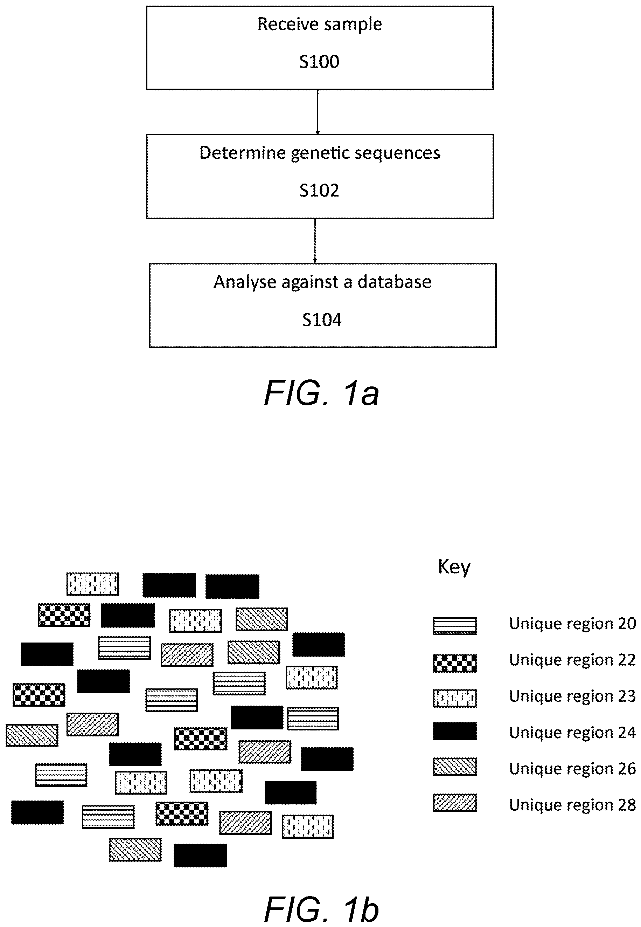

Figures 1a to 1g illustrate one possible shortcoming of such known metagenomic sequencing techniques. The basic steps of such a computational approach are shown in Figure 1a. In a first step S100, the metagenomic sample is received. As illustrated in Figure 1b, a plurality of genetic sequences 20, 22, 23, 24, 26, 28 within the metagenomic sample are then determined, e.g. read or identified, (step S102). Each of these genetic sequences is typically relatively short, e.g. between 50 to 150 bps. Each of the plurality of genetic sequences is then analysed against a metagenomics analysis database which comprises a plurality of taxonomically organised reference genomes (step S104). This analysis, termed lowest common ancestor (LCA) analysis determines the classification potential for any read sequence using tools such as Kraken or Centrifuge can be used.

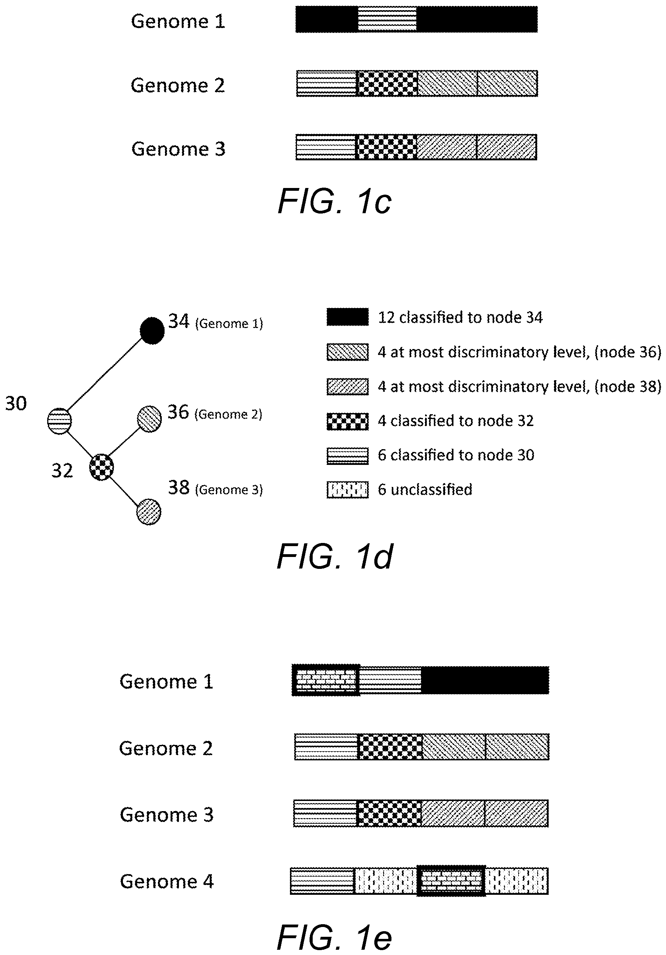

Figure 1c shows an example of contents of the database which is used for the comparison and as illustrated there are three reference genomes 1 to 3. The results of the analysis of the plurality of sequences shown in Figure 1b against this database are shown in Figure 1d. As shown in this simplified example, a first read genetic sequence corresponding to unique region 20 is shared by all three genomes and thus relates to a first branch point 30 in the tree of figure 1d. In the plurality of reads shown in Figure 1b, there are six occurrences of this unique region 20. A second read genetic sequence corresponding unique region 22 is shared by Genome 2 and Genome 3 and as such can be classified no further than to node 32. In the plurality of reads shown in Figure 1b, there are four occurrences of this unique region 22. Each of the third, fourth and fifth read genetic sequences 24, 26 and 28 are unique to regions in Genome 1, Genome 2 or Genome 3 respectively and thus relate to terminal nodes 34, 36 and 38. In the plurality of reads shown in Figure 1b, there are twelve, four and four occurrences of these unique regions 24, 26 and 28.

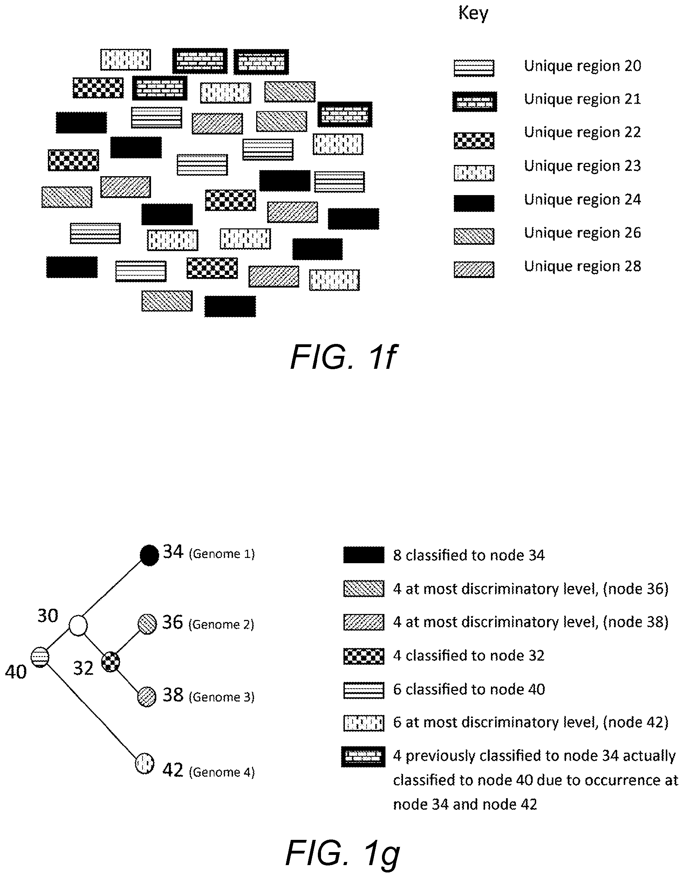

This analysis, termed lowest common ancestor analysis, determines the classification potential for any read sequence. The quality of the classification and estimations of relative abundance is fundamentally limited by the number of genomes in the database. Figure 1e illustrates an alternative database which has been expanded to include an additional species, denoted Genome 4. The inclusion of Genome 4 identifies the sequence corresponding to unique region 21 as a horizontally transferred gene/element that has been shared in recent evolutionary history and does not follow the expected phylogenetic relationship between Genome 1 and Genome 4. Previously, this sequence was identified as corresponding to unique region 24. The results of the analysis against this database are illustrated in Figures 1f and 1g. Figure 1f shows the plurality of sequences of Figure 1b with a corrected key to show the corrected identification of the reads to the unique regions. In the tree shown in Figure 1g, sequence corresponding to unique region 21 is assigned to node 40. Following the inclusion of Genome 4 in the reference genome database, four sequence segments previously assigned to unique region 24 are thus now accurately classified as sequences corresponding to unique region 21. Previously, this horizontally transferred element inflated incorrectly the relative abundance of Genome 1.

Thus, if Genome 4 is included in the database, the sequence corresponding to unique region 21 is no longer contributing to the specific identification of Genome 1 in the metagenomics sample being analysed. This is because both Genomes 1 and 4 harbour sequences to which the sequence corresponding to unique region 21 can be assigned. This means that sequence corresponding to unique region 21 would be assigned only as far as the last shared node 40 between Genome 1 and 4. Other reads would be needed to fully navigate the phylogenetic structure of the dataset to properly assess the prevalence of isolate 1 and 4 in this example. Accordingly, there is a problem with the current method of analysing the plurality of sequences, particularly when there are horizontally transferred elements.

There are other applications in which sequences are analysed and which may be inaccurate for similar reasons. For example, establishing the phylogeny of micro-organisms measures their relatedness and mapping genetic relatedness between samples or strains enables a spatiotemporal analysis of microorganism evolution. Various epidemiological studies may make use of phylogeny. For example, "Genome sequencing defines phylogeny and spread of methicillin-resistant Staphylococcus aureus in a high transmission setting" by Tong et al published in Genome Res 2015 Jan 25(1): 111-8 Epub 2014 Dec 9 describes how phylogeny may be used to identify a transmission event and trace the source of a pathogen outbreak. "Origins of the current outbreak of multi-drug resistant malaria in southeast Asia: a retrospective genetic study" by Amato et al published in The Lancet Infectious Diseases vol 18, issue 3, March 2018 describes how genome sequence data may be used to monitor the emergence of antimicrobial resistance. "Pneumococcal lineages associated with serotype replacement and antibiotic resistance in childhood invasive pneumococcal disease in the post-PCV13 era: an international whole-genome sequencing study" by Lo et al published in The Lancet Infectious Diseases vol 19, issue 7, July 2019 describes how vaccine coverage may be monitored using genomic surveillance of microorganism population in longitudinal and/or geographical studies. "Atlas of group A streptococcal vaccine candidates compiled using large-scale comparative genomics" by Davies et al published in Nature Genetics 51 p1035-1043 (2019) describes how new vaccine antigen and candidates may be identified.

WIEHLMANN ET AL, "Impact of sample processing on human airways microbial metagenomes", JOURNAL OF BIOTECHNOLOGY, vol. 250, pages 51-55, disclose a method of genomic analysis comprising whole metagenome shotgun sequencing for obtaining information about the gene content and the composition of human airways microbial metagenomes. The method comprises trimming of raw sequence reads and subsequently removing low quality reads, human reads, non-human low-complexity reads and non-human reads encoding mobile genetic elements. Masking of genetic sequences is not disclosed.

The applicant has recognised the need for more accurate, efficient and economical, high throughput metagenomic analysis or other types of genomic analysis.

### Summary

According to the present invention there is provided an apparatus, computer readable medium and method as set forth in the appended independent claims. Other features of the invention will be apparent from the dependent claims.

We describe a method for analysing a sample, the method comprising: extracting a plurality of sequence reads from within the sample; performing genomic analysis on the plurality of sequence reads by comparing the plurality of sequence reads to reference genomes stored in a reference database, wherein each stored reference genome comprises a set of reference sequences, wherein before performing the genomic analysis, the method further comprises:
comparing screening sequences with at least one of the set of reference sequences and the plurality of sequence reads from the sample; and when it is determined that a screening sequence matches at least one sequence within either the set of reference sequences or the plurality of sequence reads, masking the at least one matching sequence.

The screening sequences include genomic sequences for mobile genetic elements and/or horizontally transferred genes or DNA. A mobile genetic element may be defined as a genomic sequence which may move around within a genome. A mobile genetic element is typically functional and often comprises several genes which are mobilizable. A horizontally transferred gene is one that can be transferred from one species or replicon to another. The screening sequences may comprise plasmids, transposons, bacteriophages, genomic islands, genomic islets, integrative conjugative elements, integrative mobilizable elements, group II introns and insertion sequences. Examples are given in the description below.

The mobilisation of a piece of DNA via a plasmid or bacteriophage may require a number of biological events to take place and these are reliant on the appropriate genes being present. For example, a conjugative plasmid would require genes for replication, mobilisation and conjugal transfer. A bacteriophage requires, amongst other features, genes encoding capsid, tail and tail-fibre formation. Genes on plasmids such as antimicrobial resistance genes may be transferred without being themselves mobile elements and may thus be classed as horizontally transferable elements. Similarly, a transposon may include genes or DNA from the bacterial chromosome which is not required for transposition, but which is rather transferred as a consequence of the transposition event and may thus be classed as horizontally transferable elements.

The screening sequences may be stored in a screening database. Thus, in other words, the determining step may comprise screening against the screening sequences within the database. A match may be determined, if for example 95% of the compared sequence (i.e. a reference sequence from the reference genome or a sequence read from the sample) is identical to the screening sequence or the compared sequence is identical to the screening sequence.

The sample may be from a patient, e.g. a human or animal. The sample may also be from an animal which is a disease vector. For example, the sample may be a faecal sample and may contain a plurality of gastrointestinal microbiota. Alternatively, the sample may be a blood sample, saliva, sputum, urine or a sample from an oral swab, a vaginal swab, a wound swab and a nasopharyngeal swab. The sample may contain a plurality of micro-organisms and/or a plurality of distinct species. A sample from an environment may be a soil sample, a sea-water sample, a water-effluent sample, a food sample, a sample from a surface (e.g. a hospital bed).

Masking the matching genomic sequence comprises converting the matching genomic sequence to a sequence of characters, e.g. N, which effectively render the sequence invisible in subsequent analysis. It will be appreciated that "N" is simply one suitable example and any character which is not used in a standard genomic sequence can be used. Alternatively, masking the matching genomic sequence may comprise deleting the matching sequence.

The genomic analysis may be selected from metagenomic analysis, phylogenetic analysis and/or analysis of meta-transcriptomic data.

The method comprises comparing the set of reference sequences with the screening sequences, masking any reference sequences within the reference genomes which match the screening sequences and storing the masked reference genomes. The storing step is completed before extracting the plurality of sequence reads, i.e. the database of reference genomes with at least some masked sequences may be built before the metagenomic analysis is performed. This may result in a more efficient method because it may not be necessary to screen every read from every metagenomic sample against the screening database. This may also result in a more accurate method because a genome is typically at least 1MB whereas each read may only be around 150bp and thus false positive filtering may be reduced. After the masking, the metagenomic analysis may further comprise classifying each sequence read as belonging to a particular sequence. The classification may be termed taxonomic or phylogenetic classification. The analysis may be done by comparing each sequence read to the database of stored reference genomes, which may include stored masked reference genomes.

Alternatively, the method comprises comparing the plurality of sequence reads from the sample with the screening sequences and masking any sequence reads which match the screening sequences. This avoids the need to build a specialist database for the screening sequences which are to be masked in the sequence reads. However, it is likely to be more time consuming to screen all sequence reads against the screening sequences than performing metagenomic analysis for the sequence reads against the database of stored reference genomes. It is also more likely to be less time consuming to create the database with stored masked reference genomes as described above and perform metagenomic analysis against this database for the unscreened sequence reads.

The genomic analysis may comprise performing phylogenetic analysis for the plurality of sequence reads. The phylogenetic analysis may be performed using standard tools such as such as ssu-align, Mash, muscle, mafft, MUMmer. The phylogenetic analysis may generate one or more phylogenetic structures (also termed trees) which may be considered to be a hierarchical structure which relates genomes to each other in one or more lineages. The relationships within the hierarchical structure may be based on similarities and/or differences within the genetic sequences, e.g. genetic sequences which are present or not present may determine the relationships. The relationships may be used to give a spatiotemporal analysis of microorganisms present in the sample. Phylogenetic analysis may be particularly useful for long-read technologies because such long sequence reads may be subjected to phylogenetic analysis directly.

Performing metagenomic analysis may comprise performing lowest common ancestor reference-based metagenomic assembly analysis. The analysis may be performed against a reference database which may be a custom generated Kraken database. For example, when the sample is a faecal sample, this custom generated database may be termed the Human Gastrointestinal Microbiota Genome Collection (HGG) and may comprise a plurality of reference genomes from different sources, e.g. the Human Gastrointestinal Bacteria Culture Collection (HBC) which contains isolates from the human gastrointestinal tract and other sources.

Other types of genomic analysis may also be done. For example, transcriptomics which is used to understand gene expression in a given microbe and/or more specifically meta-transcriptomics which is used to analyse gene expression in a community of microbes, e.g. within the microbiome, using approaches such as RNAseq, may be used. The analysis may be done using the database of reference genomes with at least some masked sequences and/or by masking the sequences to be compared.

Extracting a plurality of sequence reads from within the sample may comprise using shotgun sequencing in which any DNA within the sample is broken up randomly into small segments which are then sequenced to obtain the plurality of sequence reads. The plurality of sequence reads may be obtained across the complete DNA of organisms within the sample (e.g. not just the 16S rRNA gene), e.g. whole genome shotgun sequencing.

There are various applications of the methods described above. For example, a faecal (or stool) sample may be taken from a patient with food-poisoning or any other diarrhoeal disease. Metagenomic sequencing as described above may be used to identify the pathogen causing the disease. Similarly, samples such as tracheal or lung aspirates may be used to identify the organisms responsible for chest infections by metagenomics. High-precision metagenomics may also be useful in outbreaks for epidemiological purposes, particularly to distinguish highly pathogenic strains from commensal strains of the same species (e.g. *E. coli*). For example, shotgun metagenomics generates sequence reads from the whole genome, so any biological markers of pathogenicity would also be detected and may be useful to distinguish strains of a species from each other. As another example, the relationships determined using phylogenetic analysis may be used to trace the source of a pathogen outbreak, monitor emergence of antimicrobial resistance, monitor vaccine coverage, identify new vaccine antigens and otherwise applied in genomic epidemiology. Masking the mobile elements for the purposes of phylogeny may be useful to ensure that the relationships between species or strains of micro-organisms which are present in the samples are more accurately inferred. An advantage of the method described above over traditional microbiological methods may be that the sequencing which is used provides indiscriminate species/strain identification and thus even unexpected or uncultivated species can feature.

We also describe an apparatus for analysing a sample, e.g. from a patient or environment as described above, the apparatus comprising a processor which is configured to receive a plurality of sequence reads from within the sample; and perform genomic analysis on the plurality of sequence reads by comparing the plurality of sequence reads to reference genomes stored in a reference database, wherein each stored reference genome comprises a set of reference sequences; and wherein before the genomic analysis is performed, the processor is further configured to: compare screening sequences with at least one of the set of reference sequences and the plurality of sequence reads from the sample; and when it is determined that a screening sequence matches at least one sequence within either the set of reference sequences or the plurality of sequence reads, mask the at least one matching sequence.

The apparatus may further comprise a reference database in which the reference genomes are stored and which is communicatively coupled to the processor. The apparatus may further comprise a screening database in which the screening sequences are stored and which is communicatively coupled to the processor. The reference database and/or the screening database may be local or remote from the processor. As described above, the screening sequences may include genomic sequences for mobile genetic elements and/or horizontally transferred genes or DNA. It will be appreciated that the other aspects of the method described above are also compatible with the apparatus.

We also describe a non-transitory computer readable medium comprising computer executable instructions which when executed by a computing device cause the computing device to carry out the method described above.

### Brief Description of the Figures

For a better understanding of the invention, and to show how embodiments of the same may be carried into effect, reference will now be made, by way of example only, to the accompanying diagrammatic drawings in which:
Figure 1a is a flowchart illustrating the steps in a standard process for analysing a received sample;
Figures 1b to 1g illustrate how the method of Figure 1a can lead to incorrect analysis of a plurality of genetic sequences;
Figures 2a and 2b are flowcharts illustrating the steps in the two new methods for analysing a received sample;
Figure 2c is an example of the analysis using the method of Figure 2a;
Figure 3a is a phylogenetic tree showing the diversity of the genomes within the custom database which is used in the method of Figure 2a or 2b;
Figure 3b is a graph showing the classification results for the method of Figures 2a or 2b and illustrates the percentage of classified reads for 13415 metagenomic sequenced samples split into the three most highly represented continents;
Figure 4 is a schematic block diagram of the components used in the method of Figure 2a or 2b;
Figure 5a is a graph showing the relative abundance of the dominant bacterial species, ordered by prevalence, within the 13,415 human gastrointestinal metagenomic samples;
Figure 5b is a graph showing the number of samples in which each novel species was identified and is ordered by prevalence of the species; and
Figure 5c is a DAPC analysis of functional categories showing the distinct functions associated with each dominant phylum.

### Detailed description

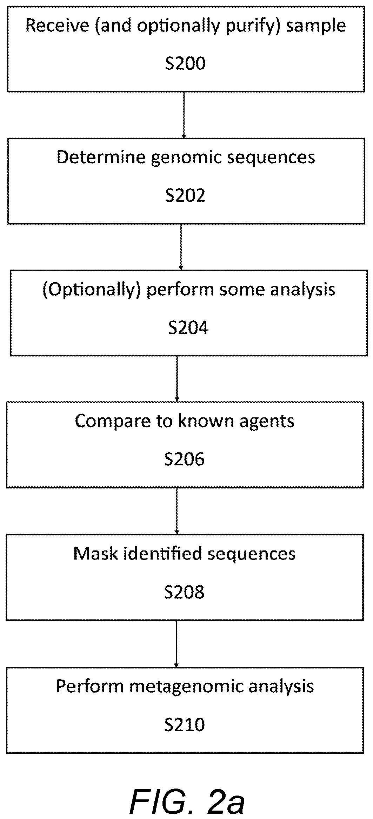

As detailed in the background section, Figures 1a to 1g illustrate a standard process for analysing a received sample and the possible incorrect analyses that may occur as a result. Figure 2a shows the steps of a proposed method which is more accurate and cost-effective shotgun metagenomics analysis.

The first step of the method (step S200) is to receive a sample. The sample may be a faecal sample, e.g. from a number (20) of healthy adults (North America n=12, United Kingdom n=8) who had not taken antibiotics within the last 6 months. Samples may be frozen and stored at -80 degrees Celsius. They may then be purified, for example using bacterial culturing as described in "Culturing of 'unculturable' human microbiota reveals novel taxa and extensive sporulation" by Browne et al published in Nature 533, 543-546, doi:10.1038/nature17645 (2016). Such culturing may use a supplemented YCFA medium with or without ethanol pre-treatment. The sample processing and culturing may take place under anaerobic conditions, e.g. in Whitley DG250 and A95 workstations at 37 °C using phosphate buffered saline and culture media incubated under anaerobic conditions for 24 hours prior to use. As an example, the faecal samples may be homogenized in reduced PBS (0.1 g stool per ml PBS), serially diluted and plated directly onto YCFA agar supplemented with 0.002 g ml⁻¹ each of glucose, maltose and cellobiose in (13.5 cm diameter) Petri dishes. Colonies may then be picked, re-streaked to purity and identified using 16S rRNA gene sequencing. Species were defined on the basis of a 16S rRNA gene sequence identity threshold of >97.8% to discriminate species for example as described in "Uniting the classification of cultured and uncultured bacteria and archaea using 16S rRNA gene sequences" by Yarza et al published in Nat Rev Microbiol 12, 635-645, doi:10.1038/nrmicro3330 (2014).

In the next step of the method (step S202), the genomic sequences within the sample may be determined. This may be done by extracting the DNA from the sample, e.g. from pelleted cells using a phenol-chloroform method as described for example in *"*Molecular cloning: a laboratory manual" 4th edn, (Cold Spring Harbor Laboratory Press, 2012). DNA may then be prepared and sequenced using standard techniques such as the Illumina Hi-Seq platform with library fragment sizes of 200-300 bp. The sequences may have a read length of 100 or 150 bp.

The genomic sequences to be used in subsequent steps may preferably be of at least high quality draft standard as defined for example in "Genome Project Standards in a New Era of Sequencing" by Chain et al published in Science 2009 October 9;326(5950): doi:10.1126/science.1180614. In other words, overall coverage represents at least 90% of the genome or target region with an effort made to exclude contaminating sequences. Sequence errors and mis-assemblies may still be possible.

Determining whether the sequences meet the standard may be achieved for example by producing annotated assemblies as described in "Robust high-throughput prokaryote de novo assembly and improvement pipeline for Illumina data" by Page et al published in Microb Genom 2, e000083, doi:10.1099/mgen.0.000083 (2016). For example, a first step in producing the annotated assemblies may be to create multiple assemblies used Velvet v1.2 and VelvetOptimiser as described in "Velvet: algorithms for de novo short read assembly using de Bruijn graphs" by Zerbino et al and published in Genome Res 18, 821-829, doi:10.1101/gr.074492.107 (2008). An assembly improvement step may then be applied to the assembly in which the best N50 and contigs were scaffolded using SSPACE described in "Scaffolding pre-assembled contigs using SSPACE" by Boezter et al and published in Genome Biol 13, R56, doi:10.1186/gb-2012-13-6-r56 (2012) and sequence gaps were filled, e.g. using GapFiller described in Toward almost closed genomes with GapFiller by Boetzer et al published in Bioinformatics 27, 578-579, doi:10.1093/bioinformatics/btq683 (2011). Automated annotation may then be performed, e.g. using PROKKA v1.11 described in "Prokka: rapid prokaryotic genome annotation" by Seemann published in Bioinformatics 30, 2068-2069, doi:10.1093/bioinformatics/btu153 (2014). In this example, genomes with less than 400 contigs, a genome size less than 8 Mb and presence of 16s rRNA sequences with greater than 97.5% homology were considered pure and included in further analysis.

Some analysis may be performed on the genomic sequences before the subsequent steps. For example, an initial phylogenetic analysis may be performed as an optional step (S204). This may be done by extracting amino acid sequences of 40 universal core marker genes from the bacterial collection, e.g. using Specl which is described in "Accurate and universal delineation of prokaryotic species" by Mende et al published in Nat Methods 10, 881-884, doi:10.1038/nmeth.2575 (2013). The protein sequences may be concatenated and aligned using standard tools such as MAFFT which is described in "MAFFT multiple sequence alignment software version 7: improvements in performance and usability" by Katoh et al published in Mol Biol Evol 30, 772-780, doi:10.1093/molbev/mst010 (2013). Maximum likelihood trees may be constructed using known techniques, such as RAxML with the standard LG model and 100 rapid bootstrap replicates for example as described in "RAxML version 8: a tool for phylogenetic analysis and post- analysis of large phylogenies" by Stamatakis published in Bioinformatics 30, 1312-1313 doi:10.1093/bioinformatics/btu033 (2014). The trees may then be visualised e.g. using iTOL described in "Interactive Tree of Life v2: online annotation and display of phylogenetic trees made easy" by Letunic et al published in Nucleic Acids Res 39, W475-478, doi:10.1093/nar/gkr201 (2011).

Screening against a screening database (step S206) may then be carried out. This screening database may comprise mobile genetic elements, for example plasmids, transposons and insertion sequences or may comprise other elements which it is desirable to remove, e.g. horizontally transferred elements (e.g. DNA encoding a specific operon). An example of a screening database is included as Supplementary Table 4 in the paper entitled "A human gut bacterial genome and culture collection for improved metagenomic analysis" by Forster et al. published in February 2019 in Nature Biotechnology.

Merely as examples, some plasmids, bacteriophages, transposons and insertion sequences are listed below using their accession numbers from the National Center for Biotechnology Information (NCBI).

| **Plasmids** | **Bacteriophage** | **Transposon** | **Insertion sequences** |
|---|---|---|---|
| AY171301 | MG711460.1 | HG475346.1 | AY168958 |
| AJ001708 | GQ141189 | U00004.1 | AF238307 |
| AY112722 | MH550421.1 | KX231277.1 | |
| X92945 | NC_004587.1 | HM243621 | |
| AM 180356 | MH382198.1 | AB426620 | |

A mobile genetic element may be a type of genetic material which can move around within a genome or that can be transferred from one species or replicon to another as described above. A horizontally transferred element is also described above. An example database comprising mobile genetic elements is known as ACLAME and comprises all known phage genomes, plasmids and transposons. The mobile genetic elements which are available in ACLAME, include plasmids, viruses and prophages. Another example database is prepared by the Millard Lab has a database of over 9000 complete bacteriophage genomes which have been extracted from GenBank. The TREP transposable elements platform is another example database which comprises transposable elements. As defined on this platform, transposable elements are mobile genomic DNA sequences found in nearly all organisms. Transposable elements have the ability to replicate in a host genome using various transposition mechanisms and they are divided into two classes based on their replication mechanism: retrotransposons (class I) and DNA transposons (class II).

Another example database is ImmeDB (Intestinal microbiome mobile element) which is a database dedicated to the collection, classification, and annotation of mobile genetic elements (MGEs) from gut microbiome. The classes of mobile genetic elements which are found in the gut microbiome may include prophages, integrative conjugative elements, integrative mobilizable elements, group II introns, transposons and genomic islands or genomic islets. Illustrative examples of some mobile elements which are provided in this database are set out below and are referenced by their ID used in the database:

| **Genomic islands** | **Genomic islets** |
|---|---|
| NZ_ADM001000031.1:84616-95833 | NC_004663.1:4599796-4601480 |
| NC_006347.1:3408664-3439675 | NZ_JH724114.1:370089-371752 |
| NZ_LRGD01000028,1:9771-24727 | NZ_JH815524.1:1786629-1788226 |
| NZ_GL945019.1:415119-429441 | NZ_NQMG01000019.1:79992-81706 |
| NC_021030.1:369579-410404 | NC_LARL01000004.1:17735-19268 |

| **Integrative conjugative elements** | **Integrative mobilizable element** |
|---|---|
| NZ_CBXG010000004.1:4988-55362 | NC_021012.1:2873017-2884015 |
| NZ_AENZ01000040.1:66378-115551 | NZ_CP011531.1:4119779-4145888 |
| NZ_BAIB02000011.1:61343-89533 | NZ_AXVN01000013.1:27402-38179 |
| NZ_CP023819.1:1567305-1600880 | NZ_JH724079.1:2703611-2729544 |
| NZ_GG774984.1:239828-297170 | NC_021040.1:960056-964689 |

| **Group II introns** |
|---|
| NZ_ADKP01000084.1:51339-53257 |
| NZ_CP022754.1:3475637-3478065 |
| NZ_JGES01000028.1:109378-117028 |
| NC_021015.1:2325689-2327578 |
| NZ_JH594448.1:1968650-1971086 |

A match may be determined when one of the sequences in the screening database and a determined genomic sequence satisfy a sequence identity condition, e.g. they are at least 95% alike, and may be preferably identical.

Once a sequence is identified as a match to a screening sequence, i.e. identified as a mobile element or a horizontally transferred element, it is then masked (step S208), e.g. by changing the sequence to a sequence of predetermined characters, e.g. Ns and effectively filtered from the plurality of read sequences which were identified in step S202. The genomics analysis is then performed at step S210 and may be metagenomics analysis or other appropriate analysis. The metagenomics analysis which is performed may be lowest common ancestor RBMA analysis which allows a determination of overall taxonomic classification efficiency across a dataset. The analysis may be performed against a custom generated Kraken database, e.g. as explained in more detail below.

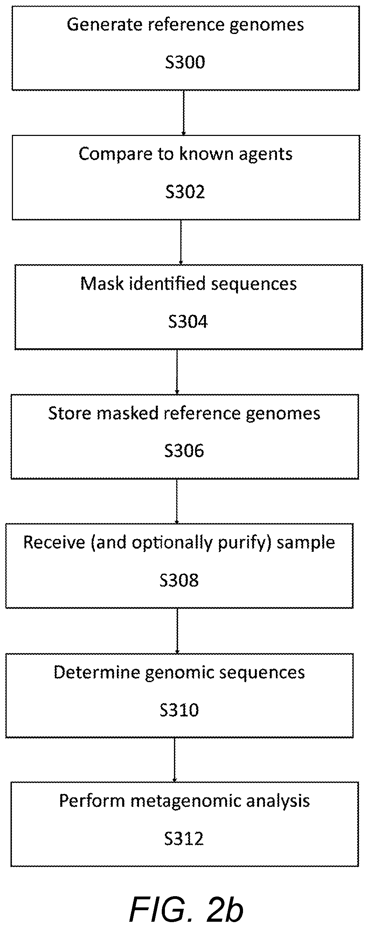

As an alternative, the screening against known elements to be masked may be used when building the custom database. Thus, as shown in Figure 2b, in a first step reference genomes for purified bacteria are generated (step S300). These reference genomes are preferably high quality draft. Each of these generated genomes is then compared to the screening database of known mobile element sequences (step S302) as described above. One method of performing the comparison is to divide each generated genome into a plurality of sequences and compare each of the plurality of sequences to the sequences within the screening database. However, it will be appreciated that other methods of identifying sequences within the reference genome which match a mobile element sequence are equally valid.

Once a sequence within the reference genome is identified as a match to a screening sequence, it is then masked (step S304), e.g. by changing the sequence to a sequence of predetermined characters, e.g. Ns. The masked reference genome is then stored together with any other reference genomes (masked or unmasked) to create the custom database (step S306). Once the database has been created, it can be used for metagenomic analysis of a sample. For example, the sample may be received and optionally purified at step S308 as in step S200 above. The genomic sequences may then be obtained as described in step S202 above. The final step of performing the genomic analysis, e.g. metagenomic analysis is then done against the database which includes any masked reference genomes.

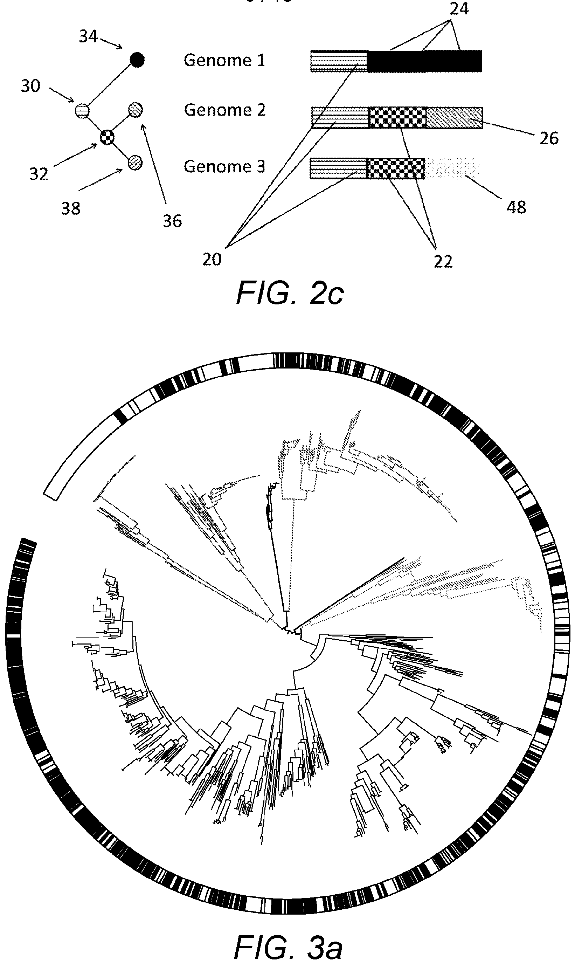

Screening and subsequently masking reference genomes as described in Figure 2b is likely to be a more efficient method and thus less time-consuming method than screening and subsequently masking the plurality of sequences in the sample as described in Figure 2a. Nevertheless, it will be appreciated that the method of Figure 2b, like that of Figure 2a, addresses the problems of incorrect analysis due to mobile elements or other similar elements which result in mis-classification of sequence reads against a phylogenetically organised reference database. Returning to the problem identified in the background, the read sequence 21 which is incorrectly assigned in Figure 1f is identified as a mobile genetic element in the reference genome using the method of Figure 2b. As schematically shown in Figure 2c, the mobile genetic element 48 is masked in the reference genome. Accordingly, any read sequences which match this region of Genome 3 are not assigned and do not result in false positive identification.

For a faecal sample, this custom generated database may be termed the Human Gastrointestinal Microbiota Genome Collection (HGG). As an example, one version of the database comprises 1354 genomes representing 530 species from 57 families within the phyla Actinobacteria (129 genomes; 55 species), Bacteroides (231 genomes; 69 species), Firmicutes (772 genomes, 339 species), Fusobacteria (26 genomes; 9 species), Proteobacteria (194 genomes; 56 species) and Synergistetes (2 genomes; 2 species). To understand the phylogenetic relationship between these taxa, we extracted 40 universal core genes from each genome and performed phylogenetic analysis which is shown in Figure 3a. The branch colour distinguishes bacterial phyla belonging to Actinobacteria (gold), Bacteroides (green), Firmicutes (blue), Fusobacteria (brown), Proteobacteria (red) and Synergistetes (black). Overall, the maximum phylogenetic diversity was observed in Firmicutes, particularly the classes Clostridia, Erysipelotrichia and Negativicutes. However, as shown in Figure 3a, a broad range of species and phylogenetic group are represented across all phyla.

This custom generated database may be created by combining data from other databases, e.g. the Human Gastrointestinal Bacteria Culture Collection (HBC) which contains isolates from the human gastrointestinal tract and other sources. Currently the HBC comprises 737 purified and archived isolates which represents 273 species (105 novel species and 173 novel genome sequences of isolates from healthy individuals) from 31 families in the phyla Actinobacteria (53 genomes; 16 species), Bacteroides (143 genomes, 40 species), Firmicutes (496 genomes, 203 species) and Proteobacteria (45 genomes; 14 species). A genome sequence is available for each isolate within the HBC. Another source which is used is the National Center for Biotechnology Information (NCBI) genome database which has 617 publicly available, high-quality human gastrointestinal associated bacterial genomes. It is noted that 53% of species in the custom HGG database have been archived in the HBC database. Many of the species currently absent from the HBC database but present in the custom HGG database include members of the Fusobacteria, Proteobacteria and Synergistetes which are typically absent from healthy individuals from the developed world.

As explained above, these genomes and the sequences against which they are compared may contain masked sequences corresponding to screening sequences contained with a reference screening database (ENI and ICEberg databases). The metagenomics samples may also be filtered by quality, e.g. using Trimmomatic which is described in "Trimmomatic: a flexible trimmer for Illumina sequence data" by Bolger et al published in Bioinformatics 30 2114-2120 doi:10.1093/bioinformatics/btu170 (2014). Human contaminating reads may also be filtered by mapping to the Human reference genome (hg19) for example using Bowtie described in "Fast gapped-read alignment with Bowtie 2" by Langmead et al published in Nat Methods 9, 357-359, doi:10.1038/nmeth.1923 (2012). Samples with fewer than one million reads after filtering may be discarded. Filtered sequences were then classified at the genus and species levels using lowest common ancestor analysis as described in "HPMCD: the database of human microbial communities from metagenomic datasets and microbial reference genomes" by Forster et al published in Nucleic Acids Res 44 D604-609, doi:10.1093/nar/gkv1216 (2016).

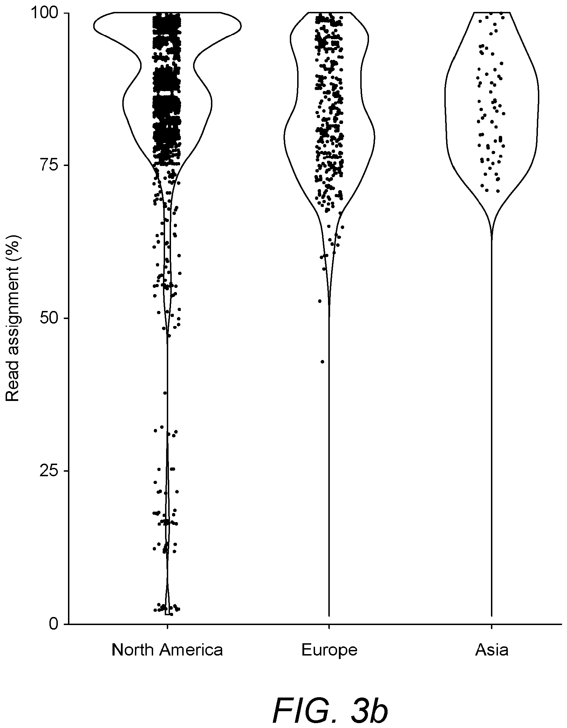

A set of results is shown in Figure 3b which illustrates the percentage of classified reads for 13415 metagenomic sequenced samples. Applying lowest common ancestor RBMA with mobile element filtering achieved an average taxonomic assignment of 83% at the genus level and 79% at species level. Taken together, these analyses reveal high resolution classification of the majority of human gastrointestinal microbiota derived metagenomics reads in each of the three most highly represented continents as shown in Figure 3b.

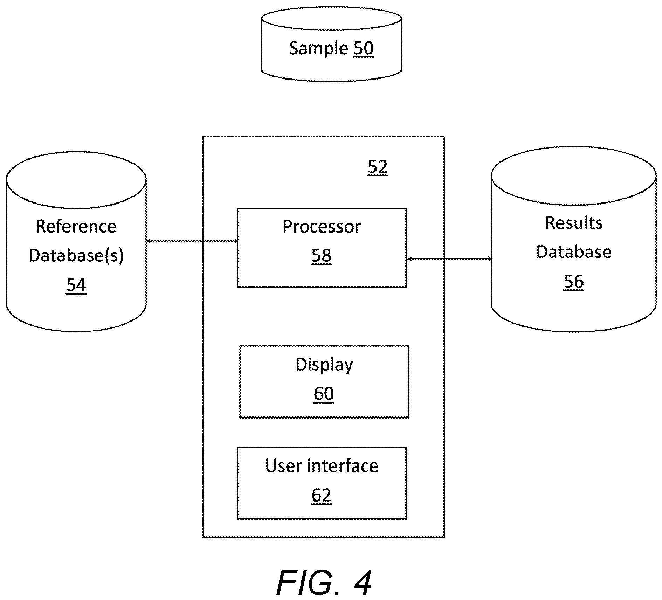

Figure 4 is a schematic block diagram showing a system which may be used to implement the method of Figure 2a or Figure 2b. The sample 50 is collected and optionally purified as described above. The sequences which are read from the sample are then input into a computing device 52 which may be a server, a stand-alone PC or any standard device which is capable of processing data. The computing device 52 may comprise standard components such as a processing module 58, a display 60 and a user interface 62. The display 60 may be used to display the phylogenetic tree to the user and the user interface 62 may be used to allow a user to input data about the sample.

The processing module 58 performs the steps of performing phylogenetic analysis, searching for incorrect families, comparing to known agents, masking identified sequences and performing the final metagenomics analysis. This module is shown as a single module but it will be appreciated that the processing steps may be split across several components, e.g. to increase processing performance. When comparing the sequence in the sample or the reference genome to known screening sequences or when comparing the sequence in the sample to reference genomes (in the RBMA analysis), the processor may access one or more reference databases 54 to which it is communicatively coupled. In this example, the reference database 54 is shown as a separate, single component but it will be appreciated that it may also be stored in memory on the computing device 52 or may be split across multiple components. In one example, the reference database 54 may be a mobile element reference database which may include 2826 plasmids and 466 transposons and insertion sequences which may be local to the process. The reference database 54 may also include a reference genome database into which the reference genomes (masked and unmasked as per Figure 2b) are stored.

The metagenomic analysis which is performed in the final step of the method may comprise generating a collection of genome sequences from the sample, e.g. bacterial isolates from a faecal sample. This collection may be stored in a results database 56 which is communicatively coupled to the processing module 58. Like the reference database 54, the results database 56 may be local or remote from the computing device 52.

As an example, the method may also be used to understand which species are most prevalent within the human gastrointestinal microbiota. Considering only species that are present at a level greater than 0.01% within any one of the 13,415 samples described above, 165 species were identified as present in more than two unrelated samples. This group of dominant species included members of the phyla Bacteroidetes (n=41), Firmicutes (n=82), Proteobacteria (n=27) and Actinobacteria (n=15). Given the background prevalence of each phylum, this represents a significant over-representation of species from Bacteroidetes (p<0.05) this represents a significant under-representation of species from Firmicutes (p<0.01).

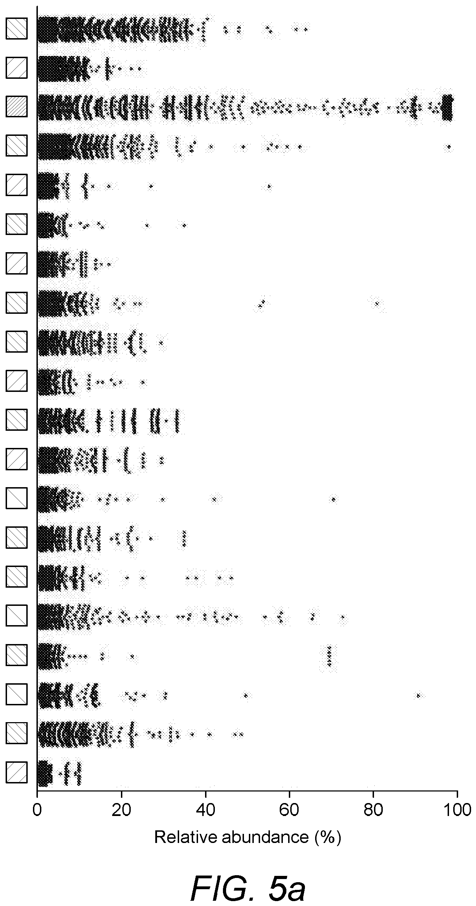

Figure 5a illustrates the relative abundance of the top 20 prevalent species within each phyla. The colour denotes Bacteroidetes (green), Firmicutes (blue), Proteobacteria (red) and Actinobacteria (yellow). Considering all species that were detected above background levels, the majority of dominant species are members of the Bacteroides genus. When corrected for the number of species within each phylogenetic group, the Bacteroidetes generally and the *Bacteroides* and *Parabacteroides* genera specifically are significantly over-represented (p<0.001). Despites over 346 species within the Firmicutes, there are only 6 distantly related Firmicutes species that were highly represented across many individuals. Overall, all detected genera within the Firmicutes phylum were statistically under-represented in their occurrence. The majority of Proteobacteria were not detected within the samples. Interestingly, no members of the Fusobacteria or Synergistetes were found to be prevalent at the level of detection considered, suggesting that they are only found during certain conditions or stages of life that were not included in this analysis.

This data suggests a potential key role for specific members of the *Bacteroides* within the human gastrointestinal tract. In contrast, the significantly greater diversity observed for species from the Firmicutes suggests a dynamic, functionally redundant group.

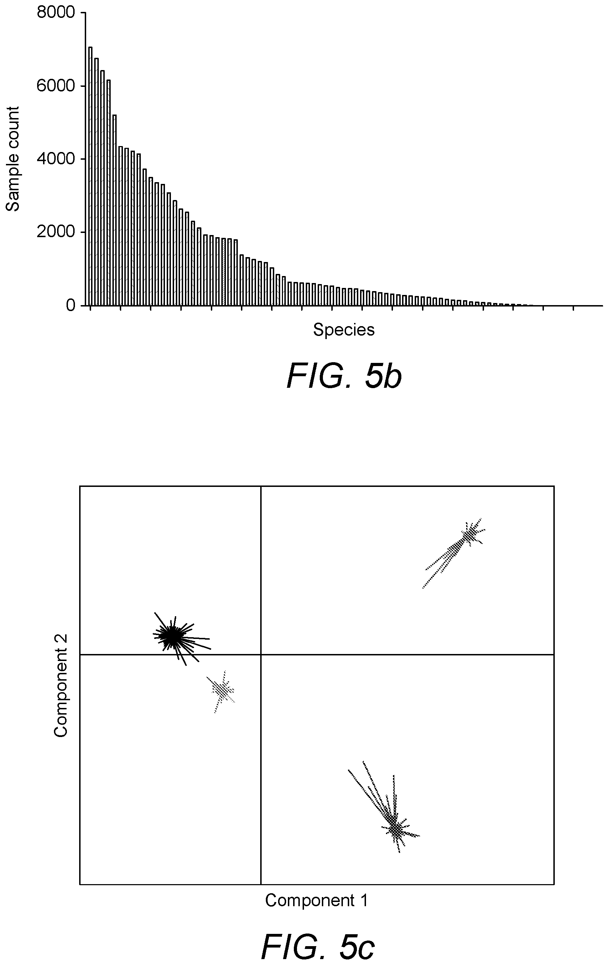

Figure 5b shows the relative abundance within a wider human cohort of the novel species which are contained within the HGG database. Importantly the availability of these genomes allows us to perform species level assessment of the prevalence of these species in metagenome samples for the first time. As shown in Figure 5b, in total 106 of the 173 novel species (60.9%) are found at greater than 0.001% abundance in at least one sample within the 13,415 public metagenome samples available. Notably almost half (87; 46%) were found in more than 100 samples but less than one quarter (389; 21.8%) were found in more than 1000 samples. Interestingly, three novel species all within the Clostridiales were found almost half the sample analysed. Two novel species *Lachnospiraceae* were found in 7797 (55.9%) and 7074 (50.7%) samples respectively and a novel *Ruminococcaceae* species was found in 6777 (48.6%) samples. This suggests that may of the novel species and genomes identified through this work occur frequently within the human population and potentially represent integral parts of the human gastrointestinal microbiota.

As shown in Figure 5c, the custom database HGG enables high resolution functional analysis in addition to detailed taxonomic analysis. This provides the capacity to enhance our understanding of the functions provided by bacteria that inhabit the gastrointestinal tract. COG analysis for example as described in "The COG database: a tool for genome-scale analysis of protein functions and evolution" by Tatusov et al published in Nucleic Acids Res 28, 33-36 (2000) is applied to identify features which are prevalent in the HGG bacteria. This analysis identified 4,696 protein domains represented in at least one isolate. As expected, bacterial housekeeping functions including ribosomal protein function, amino acid synthesis and other translation-associated functions dominate the 30 functions found in all bacteria within the collection. The 4696 protein domains which were identified were then compared using discriminant analysis of principle components (DAPC) as described in "Discriminant analysis of principal components: a new method for the analysis of genetically structured populations" by Jombart et al published in BMC Genet 11, 94, doi:10.1186/1471-2156-11-94 (2010). This comparison demonstrates clear functional differences between key phyla of the human gastrointestinal microbiota as shown in Figure 5c in which Bacteroidetes are shown in green, Firmicutes in blue, Proteobacteria in red and Actinobacteria in gold.

An enrichment analysis was then undertaken to identify those functions which were over-represented in each phylum relative to all functions present within the HGG database. This analysis identified 8, 122, 152 and 389 statistically enriched functions (q<0.001) in Actinobacteria, Bacteroidetes, Firmicutes and Proteobacteria respectively. This analysis was calculated using for example one-sided Fisher's exact test with p-value adjusted by Hochberg method. Enriched functions within the Actinobacteria were limited and primarily associated with lipid (q<1.99 x 10⁻⁸³) and carbohydrate metabolism (q<7.57 x 10 ⁻⁷⁷). Equivalent analysis of the Bacteroidetes specific functions identified many key functions, including iron (q<1.18 x 10 ⁻¹¹⁴) and sulfur transporter functions (q<6.82 x 10 ⁻⁹⁷) and specific sodium transporting NADH ubiquinone oxidoreductases (q<3.47 x 10 ⁻¹²⁴). Firmicutes were dominated by uncharacterised functions. However, spore formation (q<3.48 x 10 ⁻¹²³), thiamine (q<2.76 x 10 ⁻¹⁰¹) and riboflavin transport (q<7.04 x 10 ⁻¹⁰¹) were all highly enriched. Proteobacteria were dominated by fructose bisphosphatase (q<4.50 x 10 ⁻¹⁴⁰), glucokinases (q<4.55 x 10 ⁻¹²⁵) and regulators of iron cluster formation (q<9.20 x 10 ⁻⁹⁸). These results demonstrate the distinct differences in the unique functions provided by the key phyla of the human gastrointestinal microbiota.

The HGG collection contains novel genomes of 173 species which include HBC genomes of 105 novel species and HBC genomes of 68 species not previously isolated and genome sequenced from the human gastrointestinal tract. Analysis of these genomes to identify novel functions in the human gastrointestinal microbiota identified 45 newly described functions, of which 41 were found in the Firmicutes. While these functions were dominated by uncharacterised proteins, novel functions included those associated with tetrahydromethanopterin S-methyltransferase, present in five species, preprotein translocase, also present in five species and formaldehyde-activating enzyme necessary for methanogenesis found in four of the uncharacterised Firmicutes. This analysis also identified Type III, IV and VI secretion system components in Bacteroidetes that were previously associated with gastrointestinal Proteobacteria and Firmicutes. Equally Proteobacteria associated ABC transporters were identified in gastrointestinal Firmicutes for the first time. The prevalence of these numerous uncharacterised functions further demonstrates the need for better genome annotation and functional genomics to understand these bacteria.

A spore signature was also applied, for example as described in "Culturing of 'unculturable' human microbiota reveals novel taxa and extensive sporulation" by Browne et al published in Nature 533, 543-546, doi:10.1038/nature17645 (2016). This revealed that 83.2% of these newly genome sequenced isolates and 85.8% of the novel species are predicted to form spores suggesting an immense amount of diversity within these groups.

It is noted that mobile genetic elements and horizontally transferred elements differ from repetitive or low complexity sequences because these simply follow a pattern without a function. The fact that mobile genetic elements have function as described above means that they are unlikely to be ignored or "masked" by individuals who are interested in generating genome assemblies to understand the biology of the species harbouring these mobile elements. Their biological relevance means that they should not be masked in this context.

Repetitive DNA sequence regions are not usually reliably assembled with reads from the short-read sequencing technologies that are commonly used to generate genome sequences. Therefore, in practice, these repeat regions can be ignored for the purposes of genome assembly, without majorly compromising the interpretation of an organism's biology. Routinely ignoring or masking genetic loci that encode several genes all of which are required to confer a particular biological function would be less benign.

Moreover, DNA or genes that are horizontally transferred become apparent and are more relevant when considered in the context of sequence reads from microbial communities. In this scenario, the true biological source of the reads encoding these elements cannot be reliably assigned. This obviously complicates any *de novo* assembly approaches that would attempt to reconstruct whole genomes from the reads representing a mixed community.

Reference based read-assignment may be a solution to this problem. However, given that mobile elements can be reliably assembled from reads representing a single species and that horizontally transferred genes may not be identified as such without investigation beyond genome assembly, it may not be obvious that misassignment of reads would occur using this method. The method and apparatus described above addresses the issues with the known techniques. Furthermore, the method and apparatus may also be relevant if long-read sequence technologies replace short-read sequence technologies for metagenomics in the future.

At least some of the example embodiments described herein may be constructed, partially or wholly, using dedicated special-purpose hardware. Terms such as 'component', 'module' or 'unit' used herein may include, but are not limited to, a hardware device, such as circuitry in the form of discrete or integrated components, a Field Programmable Gate Array (FPGA) or Application Specific Integrated Circuit (ASIC), which performs certain tasks or provides the associated functionality. In some embodiments, the described elements may be configured to reside on a tangible, persistent, addressable storage medium and may be configured to execute on one or more processors. These functional elements may in some embodiments include, by way of example, components, such as software components, object-oriented software components, class components and task components, processes, functions, attributes, procedures, subroutines, segments of program code, drivers, firmware, microcode, circuitry, data, databases, data structures, tables, arrays, and variables. Although the example embodiments have been described with reference to the components, modules and units discussed herein, such functional elements may be combined into fewer elements or separated into additional elements. Throughout this specification, the term "comprising" or "comprises" means including the component(s) specified but not to the exclusion of the presence of others.

Attention is directed to all papers and documents which are filed concurrently with or previous to this specification in connection with this application and which are open to public inspection with this specification.

Although a few preferred embodiments of the present invention have been shown and described, it will be appreciated by those skilled in the art that various changes and modifications might be made without departing from the scope of the invention, as defined in the appended claims.

The invention is not restricted to the details of the foregoing embodiment(s).

## Claims

1. A method for analysing a sample, the method comprising:
extracting a plurality of sequence reads from within the sample; and
performing genomic analysis on the plurality of sequence reads by comparing the plurality of sequence reads to reference genomes stored in a reference database, wherein each stored reference genome comprises a set of reference sequences;
wherein before performing the genomic analysis, the method further comprises:
comparing screening sequences with at least one of the set of reference sequences and the plurality of sequence reads from the sample; and
when it is determined that a screening sequence matches at least one sequence within either the set of reference sequences or the plurality of sequence reads, masking the at least one matching sequence by converting the at least one matching sequence to a sequence of predetermined characters,
wherein it is determined that a screening sequence matches at least one sequence when the at least one sequence is at least 95% alike and
wherein the screening sequences include mobile genomic elements and/or horizontally transferred elements.

2. The method of claim 1, comprising comparing the set of reference sequences with the screening sequences, masking any reference sequences within the reference genomes which match the screening sequences and storing the masked reference genomes.

3. The method of claim 2, wherein the storing step is completed before extracting the plurality of sequence reads.

4. The method of claim 1, comprising comparing the plurality of sequence reads from the sample with the screening sequences and masking any sequence reads which match the screening sequences.

5. The method of any one of the preceding claims, wherein it is determined that a screening sequence matches at least one sequence when the at least one sequence is identical to the screening sequence.

6. The method of any one of the preceding claims, wherein the genomic analysis comprises performing phylogenetic analysis for the plurality of sequence reads.

7. The method of any one of the preceding claims, wherein the genomic analysis comprises performing metagenomic analysis.

8. The method of claim 7, wherein performing metagenomic analysis comprises performing lowest common ancestor reference-based metagenomic assembly analysis.

9. The method of any one of claims 1 to 5, wherein performing genomic analysis comprises performing analysis of meta-transcriptomics data.

10. The method of any one of the preceding claims, wherein extracting a plurality of sequence reads from within the sample comprises using shotgun sequencing.

11. A non-transitory computer readable medium comprising computer executable instructions which when executed by a computing device cause the computing device to carry out the comparing, masking and genomic analysis steps performed in the method of any one of claims 1 to 10.

12. An apparatus for analysing a sample, the apparatus comprising:
a processor which is configured to
receive input data on a plurality of sequence reads from within the sample;
and
perform genomic analysis on the plurality of sequence reads by comparing the plurality of sequence reads to reference genomes stored in a reference database, wherein each stored reference genome comprises a set of reference sequences;
wherein before the genomic analysis is performed, the processor is further configured to:
compare screening sequences with at least one of the set of reference sequences and the plurality of sequence reads from the sample; and
when it is determined that a screening sequence matches at least one sequence within either the set of reference sequences or the plurality of sequence reads, mask the at least one matching sequence by converting the at least one matching sequence to a sequence of predetermined characters,
wherein it is determined that a screening sequence matches at least one sequence when the at least one sequence is at least 95% alike. and
wherein the screening sequences include mobile genomic elements and/or horizontally transferred elements.

## Patentansprüche

1. Verfahren zum Analysieren einer Probe, wobei das Verfahren umfasst:
Extrahieren einer Vielzahl von Sequenzlesungen aus der Probe; und
Durchführen einer genomischen Analyse an der Vielzahl von Sequenzlesungen, indem die Vielzahl von Sequenzlesungen mit Referenzgenomen verglichen wird, die in einer Referenzdatenbank gespeichert sind, wobei jedes gespeicherte Referenzgenom einen Satz von Referenzsequenzen umfasst;
wobei das Verfahren vor dem Durchführen der genomischen Analyse ferner umfasst:
Vergleichen von Screeningsequenzen mit mindestens einer aus dem Satz von Referenzsequenzen und der Vielzahl von Sequenzlesungen aus der Probe; und,
wenn ermittelt wird, dass eine Screeningsequenz mit mindestens einer Sequenz entweder innerhalb des Satzes von Referenzsequenzen oder innerhalb der Vielzahl von Sequenzlesungen übereinstimmt, Maskieren der mindestens einen übereinstimmenden Sequenz, indem die mindestens eine übereinstimmende Sequenz in eine Sequenz von vorbestimmten Zeichen umgewandelt wird,
wobei ermittelt wird, dass eine Screeningsequenz mit mindestens einer Sequenz übereinstimmt, wenn die mindestens eine Sequenz mindestens zu 95% gleich ist, und wobei die Screeningsequenzen mobile genomische Elemente und/oder horizontal transferierte Elemente beinhalten.

2. Verfahren nach Anspruch 1, das ein Vergleichen des Satzes von Referenzsequenzen mit den Screeningsequenzen, ein Maskieren aller Referenzsequenzen innerhalb der Referenzgenome, die mit den Screeningsequenzen übereinstimmen, und ein Speichern der maskierten Referenzgenome umfasst.

3. Verfahren nach Anspruch 2, wobei der Schritt des Speicherns vor dem Extrahieren der Vielzahl von Sequenzlesungen abgeschlossen wird.

4. Verfahren nach Anspruch 1, das ein Vergleichen der Vielzahl von Sequenzlesungen aus der Probe mit den Screeningsequenzen und ein Maskieren aller Sequenzlesungen umfasst, die mit den Screeningsequenzen übereinstimmen.

5. Verfahren nach einem der vorhergehenden Ansprüche, wobei ermittelt wird, dass eine Screeningsequenz mit mindestens einer Sequenz übereinstimmt, wenn die mindestens eine Sequenz identisch mit der Screeningsequenz ist.

6. Verfahren nach einem der vorhergehenden Ansprüche, wobei die genomische Analyse ein Durchführen einer phylogenetischen Analyse für die Vielzahl von Sequenzlesungen umfasst.

7. Verfahren nach einem der vorangehenden Ansprüche, wobei die genomische Analyse ein Durchführen einer metagenomischen Analyse umfasst.

8. Verfahren nach Anspruch 7, wobei das Durchführen der metagenomischen Analyse ein Durchführen einer referenzbasierten metagenomischen Gruppenanalyse des letzten gemeinsamen Vorfahren umfasst.

9. Verfahren nach einem der Ansprüche 1 bis 5, wobei das Durchführen der genomischen Analyse ein Durchführen einer Analyse von Metatranskriptomikdaten umfasst.

10. Verfahren nach einem der vorhergehenden Ansprüche, wobei das Extrahieren einer Vielzahl von Sequenzlesungen aus der Probe ein Verwenden einer Schrotschusssequenzierung umfasst.

11. Nicht-flüchtiges computerlesbares Medium, das in einem Computer ausführbare Anweisungen umfasst, die, wenn sie von einer Computervorrichtung ausgeführt werden, die Computervorrichtung veranlassen, die vergleichenden, maskierenden und genomischen Analyseschritte auszuführen, die von dem Verfahren nach einem der Ansprüche 1 bis 10 durchgeführt werden.

12. Vorrichtung zum Analysieren einer Probe, wobei die Vorrichtung umfasst:
einen Prozessor, der konfiguriert ist zum:
Empfangen von Eingangsdaten von einer Vielzahl von Sequenzlesungen aus der Probe;
und
Durchführen einer genomischen Analyse an der Vielzahl von Sequenzlesungen, indem die Vielzahl von Sequenzlesungen mit Referenzgenomen verglichen wird, die in einer Referenzdatenbank gespeichert sind, wobei jedes gespeicherte Referenzgenom einen Satz von Referenzsequenzen umfasst;
wobei der Prozessor, bevor die genomische Analyse durchgeführt wird, ferner konfiguriert ist zum:
Vergleichen von Screeningsequenzen mit mindestens einer aus dem Satz von Referenzsequenzen und der Vielzahl von Sequenzlesungen aus der Probe; und,
wenn ermittelt wird, dass eine Screeningsequenz mit mindestens einer Sequenz entweder innerhalb des Satzes von Referenzsequenzen oder innerhalb der Vielzahl von Sequenzlesungen übereinstimmt, Maskieren der mindestens einen übereinstimmenden Sequenz, indem die mindestens eine übereinstimmende Sequenz in eine Sequenz von vorbestimmten Zeichen umgewandelt wird,
wobei ermittelt wird, dass eine Screeningsequenz mit mindestens einer Sequenz übereinstimmt, wenn die mindestens eine Sequenz mindestens zu 95% gleich ist, und wobei die Screeningsequenzen mobile genomische Elemente und/oder horizontal transferierte Elemente beinhalten.

## Revendications

1. Procédé d'analyse d'un échantillon, le procédé comprenant les étapes suivantes :
extraire une pluralité de lectures de séquences provenant de l'intérieur de l'échantillon ; et
effectuer une analyse génomique sur la pluralité de lectures de séquences en comparant la pluralité de lectures de séquences à des génomes de référence stockés dans une base de données de référence, chaque génome de référence stocké comprenant un ensemble de séquences de référence ;
dans lequel, avant d'effectuer l'analyse génomique, le procédé comprend en outre les étapes suivantes :
comparer les séquences de criblage avec au moins l'une de l'ensemble de séquences de référence et la pluralité de lectures de séquences provenant de l'échantillon ; et
lorsqu'il est déterminé qu'une séquence de criblage correspond à au moins une séquence dans l'ensemble de séquences de référence ou dans la pluralité de lectures de séquences, masquer l'au moins une séquence correspondante en convertissant l'au moins une séquence correspondante en une séquence de caractères prédéterminés,
dans lequel il est déterminé qu'une séquence de criblage correspond à au moins une séquence lorsque l'au moins une séquence est similaire à au moins 95 %, et
dans lequel les séquences de criblage comprennent des éléments génomiques mobiles et/ou des éléments transférés horizontalement.

2. Procédé selon la revendication 1, comprenant de comparer l'ensemble de séquences de référence aux séquences de criblage, de masquer toutes les séquences de référence dans les génomes de référence qui correspondent aux séquences de criblage et de stocker les génomes de référence masqués.

3. Procédé selon la revendication 2, dans lequel l'étape de stockage est achevée avant l'extraction de la pluralité de lectures de séquences.

4. Procédé selon la revendication 1, comprenant de comparer la pluralité de lectures de séquences provenant de l'échantillon aux séquences de criblage et de masquer toutes les lectures de séquences qui correspondent aux séquences de criblage.

5. Procédé selon l'une quelconque des revendications précédentes, dans lequel il est déterminé qu'une séquence de criblage correspond à au moins une séquence lorsque l'au moins une séquence est identique à la séquence de criblage.

6. Procédé selon l'une quelconque des revendications précédentes, dans lequel l'analyse génomique comprend la réalisation d'une analyse phylogénétique pour la pluralité de lectures de séquences.

7. Procédé selon l'une quelconque des revendications précédentes, dans lequel l'analyse génomique comprend la réalisation d'une analyse métagénomique.

8. Procédé selon la revendication 7, dans lequel la réalisation d'une analyse métagénomique comprend la réalisation d'une analyse d'assemblage métagénomique basée sur la référence de l'ancêtre commun le plus proche.

9. Procédé selon l'une quelconque des revendications 1 à 5, dans lequel la réalisation de l'analyse génomique comprend la réalisation d'une analyse de données métatranscriptomiques.

10. Procédé selon l'une quelconque des revendications précédentes, dans lequel l'extraction d'une pluralité de lectures de séquences provenant de l'intérieur de l'échantillon comprend l'utilisation d'un séquençage aléatoire.

11. Support non transitoire lisible par ordinateur comprenant des instructions exécutables par ordinateur qui, lorsqu'elles sont exécutées par un dispositif informatique, amènent le dispositif informatique à effectuer les étapes de comparaison, de masquage et d'analyse génomique réalisées dans le procédé de l'une quelconque des revendications 1 à 10.

12. Appareil d'analyse d'un échantillon, l'appareil comprenant :
un processeur configuré pour :
recevoir des données d'entrée sur une pluralité de lectures de séquences provenant de l'intérieur de l'échantillon ;
et
effectuer une analyse génomique sur la pluralité de lectures de séquences en comparant la pluralité de lectures de séquences à des génomes de référence stockés dans une base de données de référence, chaque génome de référence stocké comprenant un ensemble de séquences de référence ;
avant d'effectuer l'analyse génomique, le processeur est en outre configuré pour :
comparer les séquences de criblage avec au moins l'une de l'ensemble de séquences de référence et la pluralité de lectures de séquences provenant de l'échantillon ; et
lorsqu'il est déterminé qu'une séquence de sélection correspond à au moins une séquence dans l'ensemble de séquences de référence ou dans la pluralité de lectures de séquences, masquer l'au moins une séquence correspondante en convertissant l'au moins une séquence correspondante en une séquence de caractères prédéterminés,
dans lequel il est déterminé qu'une séquence de sélection correspond à au moins une séquence lorsque l'au moins une séquence est similaire à au moins 95 %, et
dans lequel les séquences de criblage comprennent des éléments génomiques mobiles et/ou des éléments transférés horizontalement.
